# EUROPEAN PATENT SPECIFICATION

(11) **EP 4 151 142 B1**
(45) Date of publication and mention of the grant of the patent: **03.07.2024**
(21) Application number: 21198264.0
(22) Date of filing: 22.09.2021
(51) Int. Cl.: A61B 5/00, G01B 5/00, G01B 11/245, G01B 11/25, G01B 21/04, A61B 5/107, G01S 17/89, G01B 11/24, G01B 5/008, G01B 11/00

(54) **PORTABLE DEVICE AND SYSTEM FOR SCANNING OBJECTS, ESPECIALLY HUMAN FIGURE**
TRAGBARE VORRICHTUNG UND SYSTEM ZUM ABTASTEN VON OBJEKTEN, INSBESONDERE FÜR MENSCHLICHE FIGUREN
DISPOSITIF PORTABLE ET SYSTÈME DE BALAYAGE D'OBJETS, NOTAMMENT DE LA FIGURE HUMAINE

(30) Priority: 20.09.2021 PL 43898621
(43) Date of publication of application: 22.03.2023
(73) Proprietor: Eduroco sp. z o.o, 90-562 Lodz (PL)
(72) Inventor: Trojnacki, Maciej, Warszawa (PL); Dabek, Przemyslaw, Lódz (PL); Pelka, Michal, Warszawa (PL); Jaroszek, Piotr, Pionki (PL); Kozak, Adam, Grodzisk Mazowiecki (PL); Mis, Piotr, Koszalin (PL); Brzostko, Barbara, Warszawa (PL)
(74) Representative: Wlasienko, Jozef

(56) References cited:
- CN-A- 109 668 519
- CN-A- 111 700 332
- US-A1- 2018 216 923
- US-A1- 2020 081 412

## Description

### FIELD OF THE INVENTION

Subject of the invention is a portable device and a system for scanning objects, especially a human figure, enabling quick, accurate and automated obtaining of three-dimensional models of object from multiple registered images and point clouds.

The solution pertains to generally automatic gathering of measurement data about the scanned objects of various sizes, fundamentally independent of its shape and construction, which allows for acquiring a 3D model of the object, especially the figure of a human, hence a complex measurement of shapes, for example, of a human body. Furthermore, the invention includes in its scope a system of 3D modelling of object, which the system for scanning objects is equipped with.

The present solution according to the invention in general pertains to the field of mechatronics and involves a connection of mechanical engineering, electrical engineering, computer engineering including information systems engineering, control engineering and robotics for designing and manufacturing a modern portable device and system for scanning objects, especially a human figure.

### BACKGROUND OF THE INVENTION

Development in the scanning field allows for the analysis of objects or the real world environment in order to gather measurement data of its shape and often also its appearance. Then, the gathered data can be used for generation of a 3D model of an object.

3D scanning can also be used for creating a digital, three-dimensional model of the object, especially a human figure. The computer model can be used in a wide variety of applications like a three-dimensional object analysis, for example in the case of a human figure, to determine the dimensions of the body.

3D model of an object can be a virtual representation of a specific object being scanned, represented in the measuring data. For example, the three-dimensional contour, shape etc. of an object can be rendered to data representing a geometric form, which can be sent and/or saved in memory. 3D model of an object can be defined by a set of points in 3D space connected with different geometrical objects such as triangles, lines, curved surfaces etc. One of the potential ways of generating a 3D model of an object is 3D scanning of an object, which may involve collecting multiple scans of an object at different angles, i.e. pictures or point clouds.

In case of scanning of an object, especially a human figure, usually the user stands in a fixed position and then the system creates body contour maps based on depth sensors, projectors and visible light cameras or lasers. These systems then use the body contour maps to extract the measurements of the user. Determining the dimensions of the scanned object, especially the human figure, includes building a model of the scanned object using certain approach to generation of a three-dimensional model of the user's body, i.e. determining its anthropometric dimensional parameters.

In light of this, an important issue affecting the scanned object, especially the human figure, is the speed of collecting data about the object while maintaining high accuracy of its representation. The measurement data are photos obtained from digital cameras, which enables generation of a 3D model using the technique of digital photogrammetry or LIDAR sensors, which allows to obtain a 3D model in the form of the point cloud. 3D scanning can take place using a wide variety of scanning sensors and technologies suitable for this purpose.

### DESCRIPTION OF THE STATE OF THE ART

In the state of the art, there are many devices available for scanning objects which use various technologies, such as a laser technology or a structured light for creating a 3D file. Some scanning devices are better suited for certain applications, for example some are suitable for close range digitization of the small objects, while others better digitize large objects from medium and long distances. Scanning devices vary in size, speed, resolution and accuracy, moreover, they can use their own proprietary software and file types.

Most devices for scanning objects are based on a laser technology. This technology consists in laser measurements of the distance from the point with specified spatial coordinates to examined points of the scanned object and determination of their position in the adopted coordinate system. As a result of scanning the object a large number of measurement points is obtained - the so-called point cloud. Based on it, a three-dimensional map of the surface of the scanned object can be created.

In the state of the art, an international patent application No. WO/2018/070697A1 titled "*3D SCANNER*" is known, which describes a 3D scanner capable of acquiring scan data from all directions while simultaneously minimizing the number of 3D scanner sensors. The scanner is characterized in that it comprises: a support frame of a rotating frame assembly located at the upper end of the frame which surrounds the periphery of the scanned target object, horizontally with respect to the ground surface; a frame rotating assembly including a motor and a power transmission unit to supply the rotational power of the motor, mounted and fixed to the support frame of a rotating frame assembly; a horizontal frame section which is horizontal with respect to the ground surface, coupled to the power supply member; a vertical frame section formed by folding one side of the horizontal frame section; a rotating frame that rotates around the scanned target object as the power transmission unit rotates; multiple sensors of the 3D scanner mounted on the vertical section of the rotating frame for the purpose of scanning the target object; and a scanning control unit that controls the motor and multiple 3D scanner sensors to generate stereoscopic information about the scanned target object.

Furthermore, in the state of the art US patent application No. US20190180492A1 titled: "*System and Method to Capture and Process Body Measurements*" is known, in which the system and the method of capturing and processing body measurement data, including body scanners, with stationary cameras arranged around the tower and rotating platform located in some distance from the cameras is presented. The body scanner is configured to capture a first set and a second set of depth images relating to the user standing on top of the rotating platform in the first and second capture times, creating three-dimensional user avatars associated respectively with the first and the second capture times and extracting the user's body measurement data related to the first and second capture times, respectively. The user interface is configured to retrieve and display and compare the body measurement data and three dimensional avatar of the first and second capture times.

There is also known US patent application No. US20190302257 titled "*Method for Creating a 3D-Model and 3D-Body-Scanner*", in which there is presented a method for creating a 3D model of an object located in front of a number of stationary sensors mounted on a mast or a carrier. The sensors include depth sensors, and the method includes rotating the object around while scanning the object with the sensors. The method includes segmentation of the body and fitting a virtual skeleton into the segmented body with anatomically valid degrees of freedom. Moreover, the invention relates to the 3D body scanner itself, which consists only of a mast or a support element on which one or several depth sensors are mounted, which allows to completely omit the other part normally needed to turn the scanned object.

In the state of the art, there is also a US patent application No. US20180137640A1 entitled "*3D Body Scanner Data Processing Flow*", where the device for scanning object is presented, which is an intelligent scanner of a body, comprising a rotating base with a scale and a series of depth sensors mounted on a mast to create three-dimensional depth images of the body, which is rotated slowly on the platform.

In the state of the art, there is also known application No. CN111700332A entitled "*Human body scanner*", which comprises a support disc for a user to stand. The human body scanner comprises a rotary assembly, which comprises a first rotating rod and a second rotating rod, one end of the first rotating rod is connected with a supporting assembly; the other end is rotationally connected with one end of the second rotating rod; the rotating assembly has a storage state and a working state; when the rotating assembly is in the storage state, an angle between the first rotating rod and the second rotating rod is the smallest, the other end of the second rotating rod abuts against the supporting assembly, when the rotating assembly is in the working state, the angle between the first rotating rod and the second rotating rod is the largest, and the other end of the second rotating rod is far away from the supporting assembly, and a camera shooting assembly is fixedly connected with the other end of the second rotating rod, and when the rotating assembly is in the working state, the camera shooting assembly can obtain the image information of the user. According to the human body scanner, when the rotating assembly is in the storage state, the occupied space of the human body scanner can be effectively reduced, and the human body scanner is more convenient to carry.

Moreover, in the state of the art there are also known commercial solutions from ESUN Display under the name of "*Twinlike 3D Body Scanner*", or the Fit3D solution under the name "*Proscanner*", which uses a rotating platform to rotate a human and performs a scan on one side, as well as a solution from Space Vision under the name "*SCUVEG4-FLEX*" and the solution from Texel called "*3D scanner Texel Portal MX*", which consists of a fixed platform and a rotating arm with two vertical columns with cameras. In addition, it is also worth paying attention here to the solution called "*Naked 3D Fitness Tracker*", which consists of a large mirror with a measuring system and a rotating platform.

The state of the art in the field of scanning devices for modelling an object, especially the figure of a human, shows that there are no intermediate and compromise solutions that allow obtaining a 3D model in a relatively short time, significantly shorter than in the case of using handheld scanners, and simultaneously at a relatively low price of the device compared to expensive, dedicated solutions characterized by high speed of operation and involving a large number of sensors.

The above-described solutions from the state of the art and more broadly solutions for scanning objects, which is not insignificant, obtain not very accurate measurements of the scanned object when trying to significantly shorten the scanning time, especially in the case of too unstructured or too complex objects, such as e.g., a human figure. Obtaining appropriately better measurement data requires extending the process of scanning the object significantly, which is important especially when scanning the human figure, where this time should, as a rule, be as short as possible due to the discomfort associated with the need to remain in a stationary pose for longer periods of time, which is particularly problematic for young children.

The vast majority of scanners available on the market has the form of a stand with the proper scanning device attached to it. Like the handheld scanners, they do not allow for the automation of the process of scanning an object, especially a human figure.

Therefore, in the state of the art there is no complementary portable device and system for scanning objects that would enable the automatic collection of measurement data of the scanned object, especially the human figure, while ensuring the appropriate speed of its scanning and maintaining the appropriate accuracy of this scanning, and thus collecting measurement data on the scanned object with appropriate quality, at the same time combining the possibility of scanning objects of various sizes, basically regardless of their shape and structure.

It should be also emphasized that the claimed solution according to the invention, in relation to the state of the art, is a more universal solution, easy to transport, while maintaining a relatively low price compared to solutions available on the market.

### SUMMARY OF THE INVENTION

The subject of the present invention is to develop a completely new solution in the form of a portable device and system for scanning objects, especially the figure of a human, which as a result should guarantee appropriate accuracy and repeatability of scanning of an object, especially a human figure, with a relatively shortened time of this scanning.

The present solution according to the invention should also provide such features and functionalities as:
- automation of the object scanning process enabling continuous operation of the device for scanning objects while reducing the necessary service by staff, or even eliminating it;
- detection of the presence of the scanned object on the device platform;
- providing access only to authorized users;
- lighting up the scanned object;
- the ability to configure the working space of the device for scanning objects for the assumed dimensions of the scanned object;
- quick assembly and disassembly of the device for scanning objects for the purpose of its transport;
- the ability to configure the parameters of the device for scanning objects by the user in terms of scanning time and the resulting accuracy and/or repeatability of the model;
- preview of the obtained model on the user mobile device;
- the ability to voice control the device for scanning objects and receive voice notifications about its work;
- remote configuration and diagnostics of the device for scanning objects;
- processing in a cloud computing to accelerate measurement data processing and obtain a 3D model faster.

In order to achieve the above goals, according to one aspect of the present invention, it provides a portable device for scanning objects, which has at least one mast, with sensor sets, connected in its lower part to a support element designed to place the scanned object above it, wherein the mast is situated substantially vertically with respect to a support element comprising a rotating arm, a platform and a body, wherein the mast is connected to a rotating arm situated horizontally with respect to the mast and connected to a body, on which the platform designed to place the scanned object on it is located, and having a supporting set and a control unit for controlling the portable device for scanning objects, wherein the mast is shaped such that it rotates with the rotating arm around the stationary platform and the stationary supporting set, wherein
at least one mast comprises at least one mast lower arm and at least one mast upper arm articulated with each other by means of an upper yoke forming a tilting structure of the mast in such a way that the mast upper arm is tilted in direction to the rotating arm with respect to the mast lower arm and mast lower arm is tilted in direction away from the rotating arm with respect to the mast upper arm, wherein the mast lower arm in its lower part is detachably connected to the rotating arm by means of a lower yoke.

Preferably, the mentioned portable device for scanning objects has a modular structure consisting of at least two modules comprising a first and second modules, wherein the first module has a mast comprising at least one mast lower arm and at least one mast upper arm articulated with each other by means of an upper yoke, and the second module has the platform with the body and the rotating arm.

Preferably the tilting structure of the mast allows the mast lower arm to be tilted in direction away from the rotating arm with respect to the mast upper arm within the range of at least from 0 degrees to +45 degrees, and the mast upper arm to be tilted in direction to the rotating arm with respect to the mast lower arm within the range of at least from -45 degrees to 0 degrees.

Preferably, the sensor set on the mast is mounted by means of a sensor mounting connector on a trolley which by means of a stepper motor moves longitudinally in a linear manner, respectively, along the mast lower arm and the mast upper arm, to obtain a given number of positions in relation to the scanned object.

Preferably, the sensor set mounted on the sensor mounting connector has the ability to adjust the tilt angle by means of a servo-drive, thus adjusting to the scanned object in the range of at least +/- 45 degrees.

Preferably, the sensor set includes sensors enabling to collect data concerning the scanned object, in particular LIDAR sensors and/or magnetic sensors and/or cameras and/or smartphones equipped with cameras.

Preferably, a presence sensor is mounted on the mast lower arm to detect the presence of the scanned object on the platform.

Preferably along the mast lower arm and/or along the mast upper arm a LED bar adjacent directly to the mast arms is integrated for lighting up the scanned object, and/or along the mast lower arm and/or along the mast upper arm there is at least one set of LED lamps not adjacent to the mast arms fixed to the mentioned arms by means of a lighting mounting connector.

Preferably the supporting set is configured in such a way that it allows to change the size of a support surface by means of extendable legs moving relative to an extendable cross arm and/or it allows to change the spacing of the legs by means of extension of the cross arm from the tubular structure, wherein the extension of the legs and/or the extension of the cross arm is blocked by means of at least one screw.

According to a further aspect of the present invention, the present invention provides a system for scanning an object, characterized in that it includes the portable device for scanning objects. This portable device for scanning objects is equipped with the following subsystems: a control subsystem C, a power subsystem P, a communication subsystem W, a sensor subsystem S and an arm drive subsystem DA, wherein the control subsystem C interacts with the power subsystem P containing DC converters and an alternating-current adapter and/or a battery AC, wherein between these subsystems C and P there is an interaction with the above-mentioned subsystems: the communication subsystem W, the sensor subsystem S and the arm drive subsystem DA, wherein the mentioned portable device for scanning objects is connected via a communication subsystem W to a wide area network WAN through which it interacts with an information system COS, external information systems EXT and user mobile devices UMD and/or an alternative system.

Preferably, the mentioned portable device for scanning objects is additionally equipped with the following subsystems: a trolley motion subsystem DT, a head tilting subsystem DH and a lighting subsystem DL, and those subsystems interact with the control subsystem C and the power subsystem P.

Preferably, the mentioned information system COS has a web application IA, a computing application CA, a database DB and a data storage AD.

Preferably, the external information systems EXT include, in particular, external payment processing systems and social media systems.

Preferably, the mentioned system for scanning objects interacts with the user mobile device UMD and/or an alternative system configured to manual control or voice control of the portable device for scanning objects and to obtain optional voice notifications and/or to preview the obtained scanned object based on a user mobile application UMA installed on the user mobile device UMD or a desktop computer.

Preferably, the mentioned system for scanning objects has the ability to switch off selected subsystems involved in generating a 3D model such that a user operating the portable device for scanning objects obtains the scanned object of lower quality correspondingly faster by using the user mobile application UMA installed on the user mobile device UMD.

The envisaged solution according to the aspects proposed above provides an appropriate device and a system for scanning objects, especially a human figure, enabling quick, accurate and automated 3D modelling of an object on condition of appropriate collection of measurement data on the scanned object, using the designed elements and systems according to the invention.

The developed solution according to the invention also has the ability to remotely set the parameters of the used sensors, e.g., in the case of cameras: depth of field, exposure time and sensitivity, by creating a module for selecting parameters of the sensors used to scan the object. The solution also has the option of turning off selected modules involved in generating the 3D model, so that in the event that we are dealing with the scanner user, he/she will be able to see the initial scan result faster. The proposed solution according to the invention is also equipped with software for removing artifacts, i.e., side effects of the operation of the 3D model generation algorithms, which are not part of the scanned object, especially the figure of the scanned person.

Thus, the proposed solution according to the invention leads to the automatic achievement of an accurate and repeatable 3D modelling of an object, especially a human figure. Obtaining such functionality is not possible in the case of devices for scanning objects available on the market, especially handheld scanners, and it is necessary when using 3D modelling of an object in such areas as, for example, printing figures, monitoring the progress of training or therapy, or selection of clothing.

The undoubted advantage of the claimed solution is that the solution according to the invention provides the generation of a 3D model with a relatively low number of errors related to changing the position of the scanned object, in particular, it concerns the scanned figure of a human, with a relatively short scanning time compared to solutions from the state of the art. This solution provides lighting up of the scanned object and also allows the detection of the presence of the scanned object on the platform of the device. Moreover, the solution ensures high mobility thanks to the modular structure of the solution according to the invention, which is enabled by its compact size and relatively low weight, making it easier to transport the device to any place. In addition, only authorized persons can access the device which is ensured by logging in to the system by the user.

### SHORT DESCRIPTION OF THE DRAWINGS

The subject matter of the invention is illustrated in the example implementation with reference to the attached drawings in which:
- **FIG. 1**: shows in various orthographic projections in top, front, side, rear and bottom views the portable device for scanning objects, in particular the human figure, according to the invention;
- **FIG. 2**: presents in the form of an axonometric projection the portable device for scanning objects in one example of implementation according to the invention;
- **FIG. 3**: presents an axonometric projection of the modular structure of the portable device for scanning objects in one example of implementation according to the invention;
- **FIG. 4**: shows in the form of an axonometric projection in the folded and ready for transport version the mast of the portable device for scanning objects in one example of implementation according to the invention;
- **FIG. 5A** - **5B**: shows the components respectively of the mast lower arm and the mast upper arm of a portable device for scanning objects in one example of implementation according to the invention;
- **FIG. 6A** - **6B**: shows the support element of a portable device for scanning objects in one example of implementation according to the invention;
- **FIG. 7**: shows angular ranges for the mast lower arm and the mast upper arm of a portable device for scanning objects in one example of implementation according to the invention;
- **FIG. 8**: shows a block diagram of the system for scanning objects in one example of implementation according to the invention, together with the portable device for scanning objects.

### DETAILED DESCRIPTION OF THE INVENTION

Below, the subject matter of the present invention is described in detail with reference to the attached Figures and examples of implementation. The present invention is not limited to the specific examples of implementation described herein.

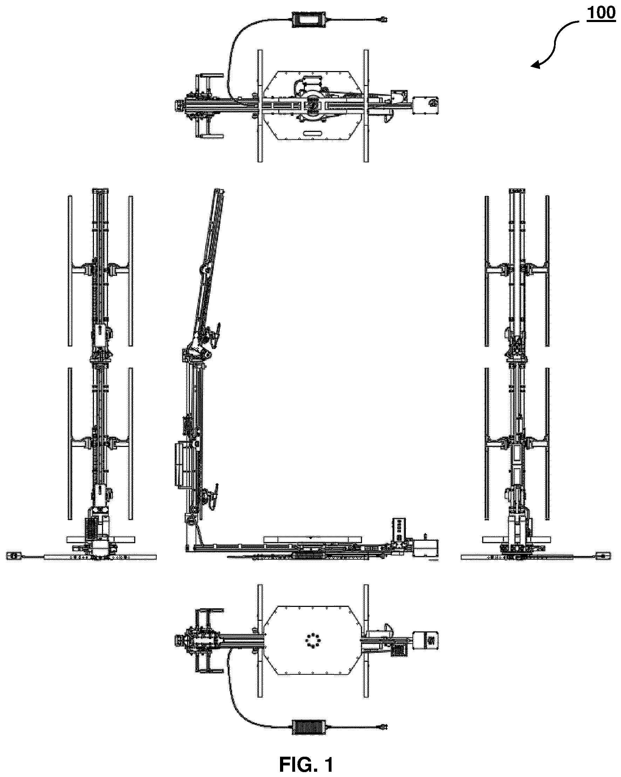

In the presented example of implementation in **FIG. 1** is illustrated in different orthographic projections in top, front, side, rear and bottom views the portable device 100 for scanning objects of various sizes, basically regardless of their shape and structure, designed to automatically collect measurement data on the scanned object, especially the human figure.

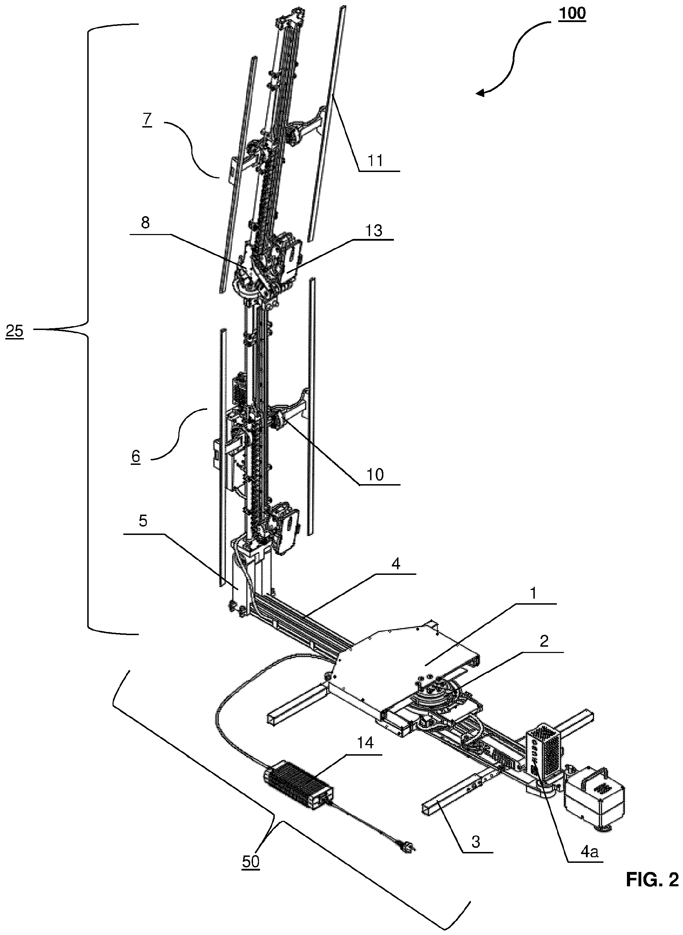

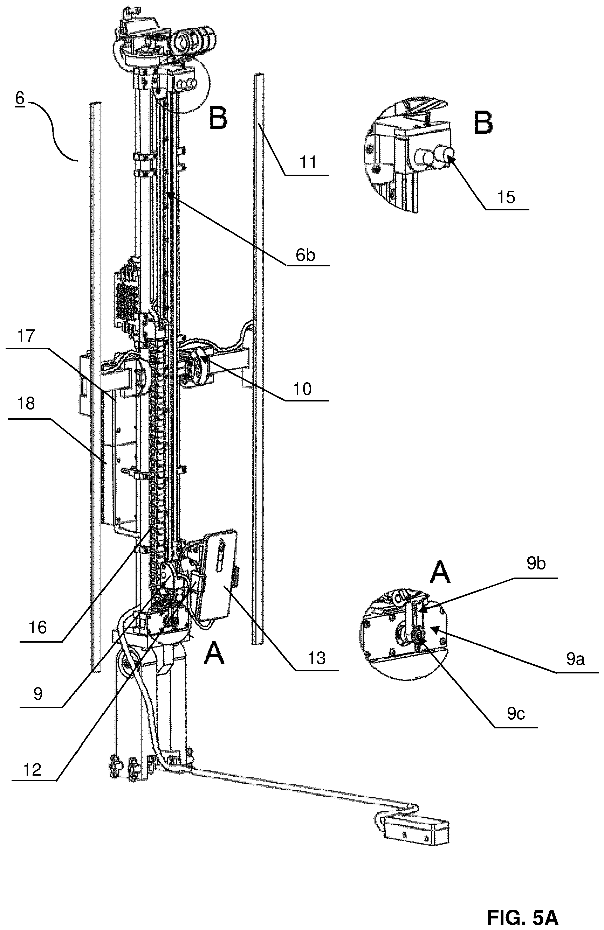

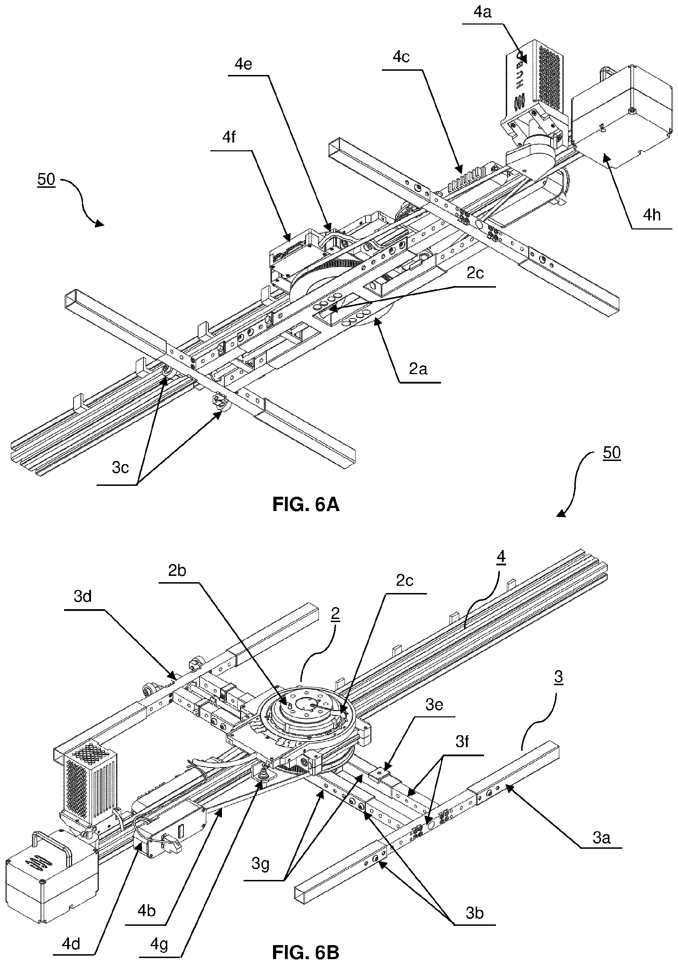

In **FIG. 2** there is shown a structural construction of the portable device 100 for scanning objects in one example of implementation according to the invention. The present device 100 for scanning objects in this example of implementation has one mast 25 situated substantially vertically with respect to the support element 50 designed to place the scanned object above it. The support element 50 comprises a rotating arm 4, a platform 1 and a body 2, wherein the mast 25 is connected to the rotating arm 4 situated horizontally with respect to the mast 25. The mentioned rotating arm 4 according to the example of implementation is connected to the body 2, on which the platform 1 designed to place the scanned object on it is located. The scanned object is above the support element 50. The stability of the structure is ensured by the supporting set 3 rigidly integrated with the body 2. The mast 25 according to the example of implementation is shaped such that it rotates with the rotating arm 4 around the stationary platform 1 and the stationary supporting set 3. In the presented example of implementation, the mentioned mast 25 comprises the mast lower arm 6 and the mast upper arm 7 articulated with each other by means of an upper yoke 8 forming a tilting structure of the mast 25. In turn, the mast lower arm 6 in its lower part is detachably connected to the rotating arm 4 by means of a lower yoke 5. For controlling the device 100 for scanning objects is responsible the control unit consisting of the primary control subsystem 17 (see **FIG. 5A**) dedicated to controlling the operation of the present device 100 for scanning objects, including controlling the sensor set 13 (including triggering photos or scans), and the secondary control subsystem 18 (see **FIG. 5A**) dedicated to controlling the drive assembly 4a and LED lamps 11, to operating the presence sensor 15, and to controlling the movement of two sensor sets 13 arranged in the example of implementation on the mast 25, one set on the lower arm 6 and the other set on the upper arm 7, respectively. In turn, for implementation of drive control of the arm is responsible the arm drive controller 4d illustrated in **FIG. 6B****.**

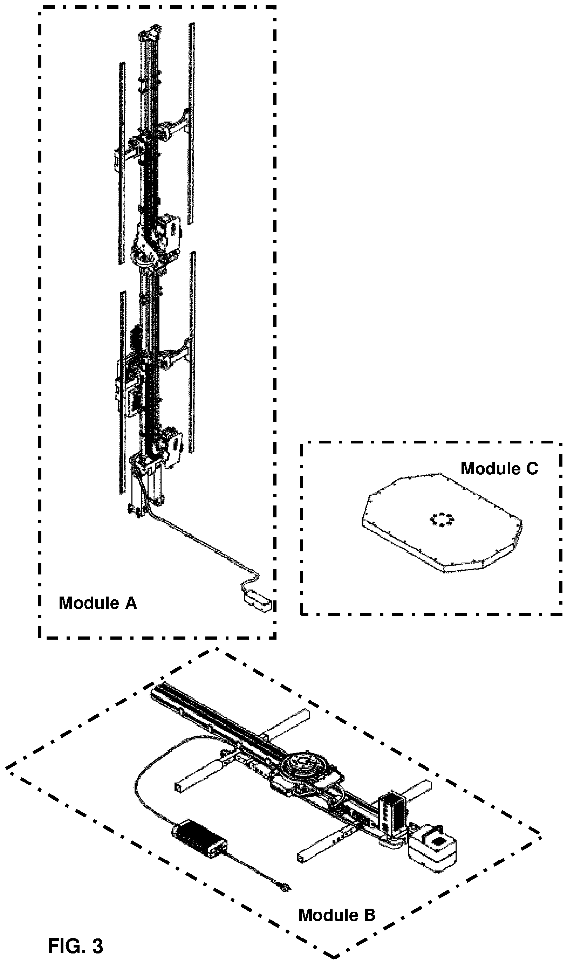

The present device 100 for scanning objects according to the above-mentioned construction has a modular structure as illustrated in more detail in **FIG. 3****,** where the modular structure illustrated according to one example of the implementation consists of three modules: a first module A, a second module B and a third module C. The first module A consists of a mast 25 comprising the mast lower arm 6 with the lower yoke 5 and the mast upper arm 7 articulated with each other by means of an upper yoke 8. The second module B consists of the rotating arm 4 together with the body 2 as well as the supporting set 3 and a AC adapter 14. The third module C, in turn, includes platform 1. By virtue of this design, sufficiently mobility of the entire device 100 for scanning objects was achieved by limiting the dimensions of the device during transport and the weight of individual modules to be carried by a human without the use of transport devices. Moreover, the presented modular structure ensures durable, but also detachable mounting of individual modules to each other according to the invention.

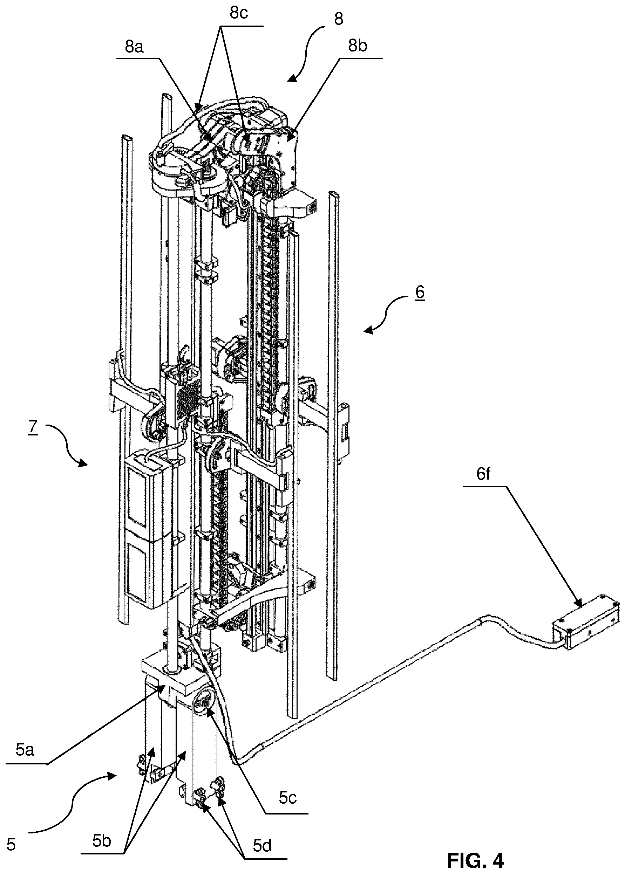

For clarity, in **FIG. 4** there is shown, in an assembled ready-to-transport version, the mast 25 constituting the module A of the portable device 100 for scanning objects. This module includes: the already defined mast lower arm 6 with the lower yoke 5 and the mast upper arm 7 connected to each other by means of the upper yoke 8 enabling mutual rotation during folding by 180 degrees of the mentioned mast arms 6 and 7 relative to each other to the folded position.

The mentioned lower yoke 5 consists of a lower part 5b of the lower yoke which is connected detachably with the rotating arm 4 (see **FIG. 2**) and the upper part 5a of the lower yoke connected to the lower arm 6 (see **FIG. 2**). Both parts of the lower yoke 5, i.e., the parts 5a and 5b, can rotate relative to each other and this rotation is blocked by two screws 5c on both sides of the lower yoke 5. The lower part 5b of the lower yoke can, in turn, slide freely along the rotating arm 4. This sliding can be blocked by four screws 5d located on both sides of the lower yoke 5, allowing the lower yoke 5 to be fixed in a desired position on the rotating arm 4. The present solution thus makes it possible to fix the lower arm 6 to the rotating arm 4 at a desired distance from its rotation axis. It also allows to set the desired tilt angle of the lower arm 6 in relation to the rotating arm 4. The mentioned upper yoke 8, in turn, consists of a lower part 8a of the upper yoke connected to the lower arm 6 (see **FIG. 2**) and of an upper part 8b of the upper yoke connected to the upper arm 7 (see **FIG. 2**), thus connecting the lower arm 6 with the upper arm 7 in a way that allows for mutual rotation of arm 6 and arm 7 of the mast. Both parts of the upper yoke, i.e., parts 8a and 8b can rotate relative to each other and this rotation is blocked by the screw 8c. This solution, in turn, allows the arms 6 and 7 to be folded for transport and to set a desired angle between these arms when scanning the object.

The proposed structure ensures quick folding and unfolding of the mentioned mast 25 of the device 100 for scanning objects, which is not without significance for the present solution. Of course, this does not exclude the possibility of defining the modular structure otherwise, in the form of other components detachably connected to each other or allowing the individual components to be assembled in a different manner.

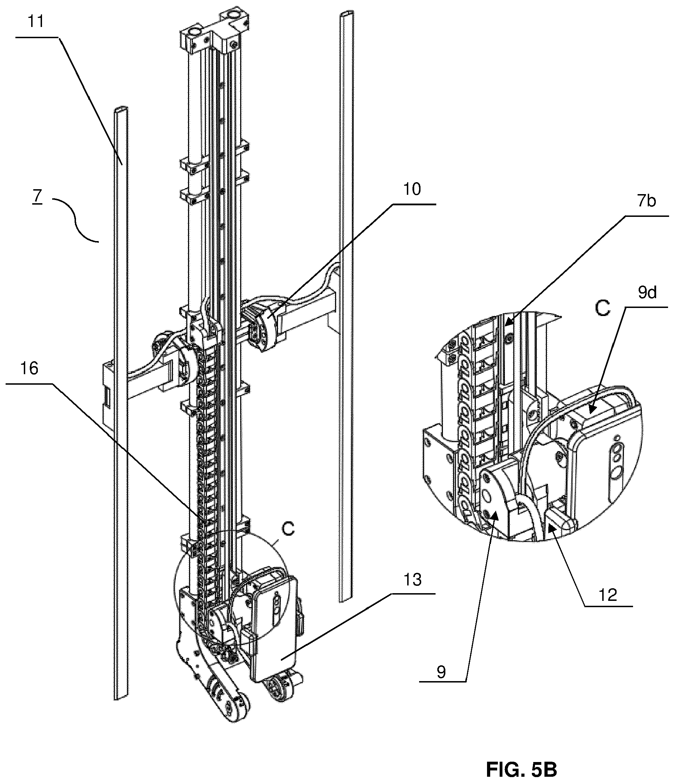

In **FIG. 5A** and **5B** there are shown the components of the lower arm 6 and the upper arm 7 of the portable device 100 for scanning objects, respectively. In this example of implementation, the mast lower arm 6 and the mast upper arm 7 include one sensor set 13 mounted to the trolley 9 via a sensor mounting connector 12. The trolleys 9 automatically move, one on the trolley guide rail 6b and the other on the trolley guide rail 7b, respectively. The trolleys 9 are moved longitudinally in a linear manner along the mast lower arm 6 and the mast upper arm 7, respectively, to obtain a desired number of positions of the sensor set 13 with respect to the scanned object. The present trolley 9 is put in motion by the stepper motor 9a and via the drive transmission unit which includes a toothed belt 9b with a gear 9c which is illustrated in more detail in an enlarged manner in **FIG 5A** in view A for the lower arm 6 - an analogous solution was used for the upper arm 7. For the movement of the trolley 9 is responsible the secondary control subsystem 18 situated on the mast lower arm 6. Importantly for the present trolley 9, it is integrated with the cable carrier 16. The cable carrier 16 ensures that the cables are secured during the movement of the mentioned trolley 9, on the trolley guide rail 6b of the mast lower arm 6 and the trolley guide rail 7b of the mast upper arm 7, respectively.

In another variant of implementation, the range of movement of the trolley 9 on the mast lower arm 6 and the mast upper arm 7, respectively, and the tilt of the sensor set 13 are predetermined for the maximum assumed height of the scanned object, in particular the human figure. In another variant of implementation, the range of movement of the trolley 9 and the tilt of the sensor set 13 by means of the servo-drive 9d are automatically adjusted to the height of the scanned object, especially the human figure which, in the example of implementation, is achieved prior to starting the proper scan by performing a single measurement and rapid processing of that measurement data to establish a desired height of the scanned object. In an alternative variant of implementation, in the case of the scanned object, which is the scanned person, one may enter one's own height in the mobile application. It is also worth emphasizing here that the scanning range of an object can also be narrower, i.e., it may cover a part of the scanned object, especially of the human figure. Thus, it can be assumed that the scanning range can be adjusted automatically to the scanned object, e.g., human height for full body scanning, or it can be limited to a user defined range.

In another variant of implementation, the mast lower arm 6 and the mast upper arm 7 each comprise three sensor sets 13 permanently mounted on the mast lower arm 6 and the mast upper arm 7, respectively. In this case, it is only possible to manually change the position of the mentioned sensor set 13 on the mast lower arm 6 and the mast upper arm 7, respectively.

In the realized example of implementation, the sensor set 13 mounted on the sensor mounting connector 12 has the ability to adjust the angle of tilt by means of a servo-drive 9d, thus adjusting to the scanned object in the range of +/- 45 degrees. The sensor set 13 consists of sensors that allow for collection of data about the scanned object and they may be magnetic sensors and/or cameras and/or smartphones equipped with cameras, as well as LIDAR sensors. In the realized example of implementation, the sensor sets 13 are smartphones equipped with cameras.

In a more preferable variant of implementation according to the invention, along the mast lower arm 6 and along the mast upper arm 7 the lighting in the form of a LED bar is integrated for lighting up the scanned object (which has not been illustrated for clarity of the presented Figures). In another variant of implementation, the lighting of the scanned object is a set of LED lamps 11, which is located along the mast lower arm 6 and along the mast upper arm 7 in a way not adj acent to these arms, attached to them by means of a lighting mounting connector 10. The purpose of the optional LED lamps 11 is to ensure the appropriate lighting of the scanned object, when external lighting is not sufficient. Their tilt and change of position are controlled via the lighting mounting connector 10.

In the example of implementation (see **FIG. 5A** view B) on the mast lower arm 6, in its upper part, there is also a presence sensor 15, detecting the presence of the scanned object on the platform 1. The present presence sensor 15 is an ultrasonic sensor.

In turn, in **FIG. 6A-6B** there is shown a support element 50 of the portable device 100 for scanning objects in the example of implementation according to the invention. The present support element 50 includes the following components: rotating arm 4 with a body 2, supporting set 3, platform 1 and AC adapter 14 (see **FIG. 2**).

The mentioned rotating arm 4 is set in motion by means of a drive assembly 4a mounted on this arm and via a toothed belt 4b that cooperates with a gear 2a fixed to the bushing 2b. At the end of the rotating arm 4 a counterbalance 4h is attached, which contains a mass to counterbalance the mass of the mast 25, thereby preventing the device 100 from tipping over towards the mast 25.

Power for the device 100 for scanning objects in one example of implementation is supplied by an AC adapter 14 (see **FIG. 2**), used to supply all subsystems of the device with a voltage of 230 Vac / 24 Vdc. The AC adapter 14 is connected to the power connector 3d, and then power is transferred via the cable 2c through the rotary joint connector, fixed to the bushing 2b, to the power distribution board 4c. The rotary joint connector (the detailed construction of which is not shown in the figures) enables unlimited rotation of the rotating arm 4 together with the mast 25 in relation to the stationary platform 1, the body 2 and the supporting set 3. The power distribution board 4c is connected to the arm drive controller 4d controlling the drive of rotating arm 4. Along the rotating arm 4, from the power distribution board 4c to the arm drive controller 4d the cables are routed in the form of a wire harness 4e. The wire harness 4e ends with a wire connector 4f which is connected to a wire connector 6f (see **FIG. 4**) to provide power to the subsystems located on the mast arms 6 and 7 and to communicate with them. In an alternative example of implementation, power to the device 100 for scanning objects is supplied from a battery mounted on the device 100 for scanning objects.

The supporting set 3, which is another component of the support element 50, includes two wheels 3c which can be used during transport of the support element 50 of the device 100 for scanning objects, and the four legs 3a. The structure of the supporting set 3 allows for both the extension of the legs 3a, i.e., their displacement in relation to the cross arm 3f, and the change of the spacing of the legs 3a, thanks to the moving of the cross arm 3f out of the tubular structure 3g. The extension of the legs 3a and the extension of the cross arm 3f is blocked by screws 3b. A magnet 3e is attached to the upper part of the supporting set 3, which cooperates with the magnetic field sensor 4g, thus enabling the determination of the zero angular position of the rotating arm 4.

The structure of the platform 1 in one example of implementation is made of steel profiles and aluminium covers to ensure adequate stability for placed scanned objects of various sizes and weights, essentially independently of their shape and structure.

The above-described portable device 100 for scanning objects further has the ability to configure the working space of the present device to an assumed maximum size of the object to be scanned. In the realized example of implementation, the maximum height of the scanned object is 220 cm, which is carried out by fixing the mast lower arm 6 and the mast upper arm 7 with the lower yoke 5 at a predetermined distance from the axis of rotation of the rotating arm 4, which is equal to 80 cm ±20 cm.

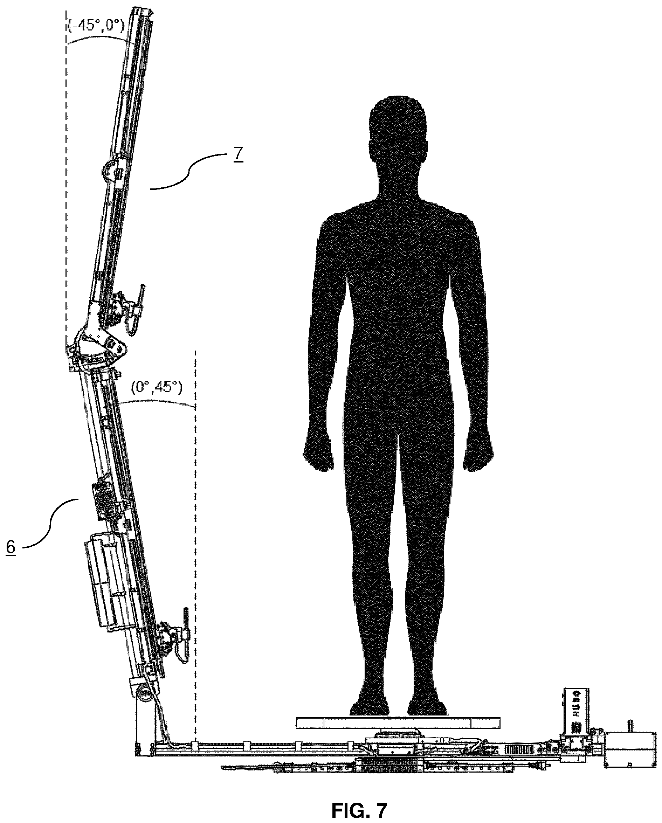

In turn, the setting of the desired joint angles in the lower yoke 5 ranges from 0 degrees to +45 degrees, and in the upper yoke 8 it ranges from 0 degrees to -45 degrees, which allows to obtain desired angles of tilt of the arms 6 and 7 of the mast, wherein the angle is measured from the vertical, as shown in **FIG. 7** illustrating the angular ranges for the above-mentioned arms 6 and 7 of the mast. A positive angle means the tilt of the mast lower arm 6 from the rotation axis outward of the device 100 for scanning objects. In turn, a negative angle means the tilt of the mast upper arm 7 from the rotation axis inward of the device 100 for scanning objects.

The above-mentioned upper yoke 8 and lower yoke 5 thus form the tilting structure of the mast 25, which enables tilting of the arms 6 and 7 of the mast and changing their distance from the axis of rotation to enable scanning of large objects with a complex geometric structure. Due to the articulated structure of the mast 25, it is possible to obtain a correspondingly better quality of the scanned object, because one looks at the scanned object slightly from above and from below, which in turn improves the quality of the obtained measurement data. The mentioned tilting structure allows the mast lower arm 6 to be tilted in the range from 0 degrees to +45 degrees, whilst the mast upper arm 7 in the range from - 45 degrees to 0 degrees, as mentioned above.

In the example of implementation according to the invention, the scanned object remains stationary and the device 100 for scanning objects by rotating the mast 25 scans the object from different sides, i.e., for the given poses of the sensor set 13 and the rotating arm 4. In one example of implementation, the scanning consists of taking pictures at particular poses of the sensor set 13 around the scanned object. According to one example of implementation, the desired poses of the sensor set 13 are obtained by tilting them by means of the sensor mounting connector 12, and moving them in relation to the mast arms 6 and 7 by means of the trolleys 9 described above in this description.

### Description of an example of operation of a scanning device

The device 100 for scanning objects is ready for operation once all subsystems have been powered on. In the example of implementation, prior to scanning an object, the mentioned device 100 performs a one-time homing process. This process consists in moving the rotating arm 4 together with the mast 25 from its current position to the zero position, which is always associated with rotating the mast 25 about the vertical axis until the zero position is detected by the magnetic field sensor 4g.

The process of scanning an object using the device 100 for scanning objects relies on gathering the data on the scanned object from the different positions of the sensor sets 13 around the scanned object, wherein the nature of the collected measurement data may be geometric, visual or other, depending on the type of used sensor sets 13, and it proceeds as follows:
- the mast 25 with the rotating arm 4 of the device 100 for scanning objects rotates in relation to the platform 1, moving to the successive predetermined angular positions;
- the device 100 collects data at successive angular positions of the rotating arm 4, wherein in one variant of implementation, the trolleys 9, on which the sensor sets 13 are placed, can additionally move in each position, which allows for obtaining different positions of the sensor sets 13 with respect to the mast 25.

The above process is carried out until the entire object scanning process is completed, i.e., until the measurement data for all desired positions of the sensor sets 13 along the arms 6 and 7 are acquired.

In view of the above, depending on the scanned object, i.e., its size, shape and structure, and therefore the complexity of its structure, the angular velocity is assumed to be in the range of 1-4 rpm. As a rule, the angular velocity can be adapted to the scanned object. It is possible to obtain a relatively high accuracy of the model by using a longer scanning time and collecting more measurement data, as well as obtaining a lower quality with a shorter scanning time and a smaller amount of measurement data. It is also possible to obtain a high-quality model in a short time thanks to the simultaneous use of more measuring sensors during scanning.

In the example of implementation, using the sensor set 13 in the form of sensors of a type of cameras embedded in smartphones, a complete scan of an object with a complex geometric structure using the present device 100 for scanning objects is performed in approximately 2 minutes with four full rotations of the rotating arm 4 with the mast 25. On the other hand, for an object with a non-complex geometric structure, using the present device 100 for scanning objects, scanning is carried out in about 60 seconds with two full rotations of the rotating arm 4 with the mast 25.

Nevertheless, in the future new types of sensors may appear which in some application (e.g., medical or for creating animations in games) will be advantageous, but will require a lower or higher velocity of movement of the rotating arm 4 together with the mast 25 or the placement of more of these sensors on the mast 25 to obtain accurate measurement data.

In one example of implementation, the sensor set 13 is a camera built into, for example, a smartphone and is mounted on a movable mast 25 with the structure defined above, wherein the mast 25 rotates around the scanned object while preprocessing the photos as the object is being scanned, using the photogrammetry technique. A key feature for measuring accuracy when using this type of sensor set 13 is the number of pictures and their quality, that is, if they are properly lit and sharp (not blurred due to movement) and have the appropriate resolution. In the example of implementation, a series of 256 photos is taken using the high-resolution sensor set 13, wherein the photos are taken around the scanned object every 11 degrees, i.e., in 32 positions, with the even distribution of photos vertically. As a rule, photos are taken at right angles to the scanned object, i.e. to the axis of rotation of the rotating arm, but to obtain a correspondingly better quality of the scanned object, one should look a bit from the top and bottom at the scanned object, which is made possible by the claimed structure of the portable device 100 for scanning objects by using the tilting structure of the mast 25 and the use of different tilt of the sensor sets 13 in their different positions with respect to the mast 25. The subject device 100 also provides a complete from each side model of the scanned object including its top, and the ability to adjust the parallax, i.e., the angle at which the same part of the object is seen in two different images (photos), which affects the accuracy of the obtained 3D model.

The present solution thus allows the device 100 for scanning objects to be configured for the purpose of determination of the model of the scanned object, i.e., adjusting it to the target application of this model, which is related to the collection of appropriate measurement data concerning the scanned object - the appropriate number of photos around the scanned object using cameras built in, for example, a smartphone or in another variant of implementation the point cloud using LIDAR sensors. It is also possible to use several types of sensors simultaneously in one sensor set 13, e.g., a LIDAR sensor and a camera.

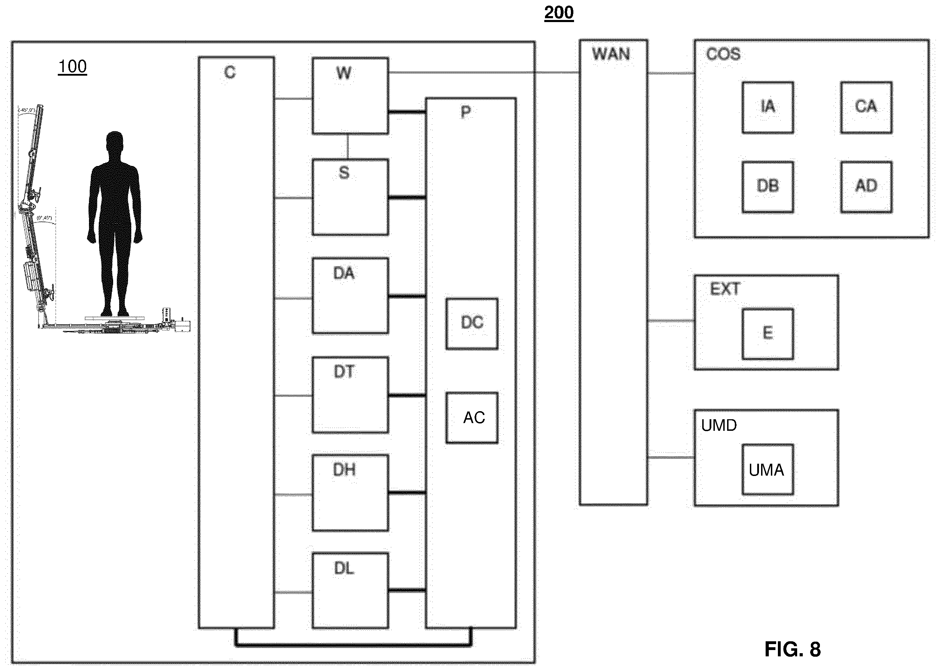

In **FIG. 8** a block diagram of the system 200 for scanning objects is shown in one example of implementation of the present invention together with a portable device 100 for scanning objects. The mentioned system 200 for scanning objects interacts with the user mobile application UMA on the user mobile device UMD, with the information system COS and also optionally with external information systems EXT providing services, e.g., payments. System 200 for scanning objects including COS, UMD and EXT communicate with each other via wide area network WAN. Depending on the industry in which the scanning system will be used, in place of the user mobile application UMA on user mobile devices UMD, e.g., a desktop application for a PC type computer may be used.

The device 100 for scanning objects includes a power subsystem P, a sensor subsystem S, a control subsystem C, a communication subsystem W, in particular wireless communication, an arm drive subsystem DA, a lighting subsystem DL, a trolley motion subsystem DT, and a head tilting subsystem DH. The trolley motion subsystem DT and the head tilting subsystem DH, which includes the sensor set 13, are optional elements and may be completely or partially omitted, e.g., in example of implementation of the device 100 for scanning objects, where the sensor set 13 is fixed directly on the mast 25 of the device 100 for scanning objects.

The diagram shows the connections between the subsystems that are specifically responsible for power supply, communication with the WAN, and local communication within the device 100 for scanning objects.

The power subsystem P supplies all components and subsystems with the appropriate voltage, either directly or via DC / DC converters (DC). The source component of the power subsystem P may be a battery or an AC adapter (illustrated in **FIG. 2** in the example of implementation as component 14) connected to the mains supply.

The sensor subsystem S comprises a sensor set 13, in which the sensors may in particular be cameras, cameras embedded in smartphones or LIDAR sensors. In the realized example of implementation, cameras embedded in smartphones are used as sensors. The number of sensors for each mast 25 of the device 100 for scanning objects mast may be between 1 and 12 depending on the type of sensor set 13 and the variant of the system 200 for scanning objects. The sensor subsystem also includes at least one presence sensor 15 that detects the presence of an object in the scanning area. Measurement data from sensors are transferred to the control subsystem C via wired or wireless communication. Moreover, the selected measurement data is transferred using the communication subsystem W and via the wide area network WAN to the information system COS.

The control subsystem C includes computer resources responsible for data acquisition, coordinating the data collection process, and coordinating the operation of the entire system of the device 100 for scanning objects. In the example of implementation, it comprises a primary control subsystem 17, responsible for the operation of the entire system, including the operation of sensor sets 13, and a secondary control subsystem 18, which implements control commanded by the primary control subsystem 17. Depending on the sensors used in the sensor set 13, the primary control subsystem 17 may also be responsible for the computations related to the determination of the 3D model. The control subsystem C transfers by means of the primary control subsystem 17 the desired motion parameters to the secondary control subsystem 18. It, in turn, transfers control to the arm drive subsystem DA, which, in the example of implementation, includes an arm drive controller 4d, and optionally to the trolley motion subsystems DT and the head tilting subsystem DH, and obtains feedback from these subsystems. The control subsystem C also transfers the desired lighting parameters to the lighting subsystem DL, which in the example of implementation includes LED lamps 11. In the example of implementation, the LED lamps 11 are directly controlled by the secondary control subsystem 18. The control subsystem C also cooperates with the information system COS, exchanging data with it and, through it, with user mobile applications UMA that operate on user mobile devices UMD or with other information systems, e.g., with a desktop application on a PC.

The communication subsystem W, in particular the wireless one, provides the control subsystem C with access to the wide area network WAN. This connection can be made through a connection to a local area network access point in the surroundings of the system 200, e.g., by means of a network card, in particular wireless one, either via a router or via a cellular modem. If the router is used, it can additionally create a local WiFi network of the scanner, which enables the operation of the sensor subsystem S, when the role of sensors is played by e.g., smartphones, and can additionally secure the information system against external access.

The arm drive subsystem DA consists of a drive assembly 4a (which includes a motor, a motor shaft rotation angle sensor or an absolute arm rotation sensor) and an optional sensor, in particular a magnetic field sensor 4g, for resetting the angular position of the arm of the device 100 for scanning objects in the absence of an absolute arm rotation angle sensor. The arm drive subsystem DA is responsible for the movement of the rotating arm 4 and is controlled by its arm drive controller 4d.

The lighting subsystem DL, controlled by the secondary control system 18, includes lamps for the mast lower arm 6 and the mast upper arm 7. The lamps may in particular be made of LED light emitting diodes of different wavelengths, including the infrared spectrum, depending on the requirements of the used sensor set, as shown in the example of implementation in the form of LED lamps 11.

The trolley motion subsystem DT is optional and serves to move the sensor set 13 along the lower and upper parts of the mast 25. The trolley motion subsystem DT includes drive assemblies, which include: a stepper motor 9a and limit sensors. This subsystem is controlled by the secondary control subsystem 18.

The head tilting subsystem DH is optional and is used to tilt the sensor sets 13 by means of servo-drives 9d depending on their position along the length of the mast 25 and the tilt angle of the mast 25 itself. This subsystem is controlled by the secondary control subsystem 18.

The integral part of the system 200 for scanning objects is the information system COS, which provides, for example, the ability to automatically obtain 3D models of scanned objects, the ability to support multiple users with ensuring access security and the ability to service a fleet of devices 100 for scanning objects. The information system COS consists of the web application IA module, computing application CA module, database DB module and data storage AD module. These modules operate as part of the IT infrastructure that enables immediate adjustment of the supply of services to the demand of users. The web application IA is the link between all major components of system 200 for scanning objects.

The database DB stores the data necessary for the operation of web applications (especially licenses and user accounts) and computing servers, as well as data related to the scan results and target 3D models.

The computing application CA is responsible for processing the data obtained during the scanning in order to obtain the final 3D models of the scanned objects.

The data storages AD are responsible for long-term storage of binary files necessary for creating models and of selected multimedia files.

Optional external information systems EXT, including external services E, e.g., payment systems, social media systems, external 3D model provider systems, can be connected to the information system COS.

System 200 for scanning objects is ready for operation when all subsystems are turned on. In the example of implementation, the device 100 for scanning objects before scanning an object performs the homing process once, which was already defined above. After collecting the measurement data, they are processed into a 3D model of the scanned object with an algorithm adequate to the type of measurement data (i.e., the type of used sensor set 13), e.g., photogrammetry techniques, SLAM, other, using the computing server CS. After completing the calculations, their result in the form of a model is sent to the user mobile application UMA installed on the user mobile device UMD or to another system, e.g., a desktop application installed on a PC, where the user can view the scanned object. If the industry of using the scanning device 100 for scanning objects requires it, for the sake of security, the measurement data are deleted from the server at the moment the service is provided. Further, the user mobile device UMD is configured to manually control or voice control the device 100 for scanning objects and to receive optional voice notifications.

An integral part of the system 200 for scanning objects is the user mobile application UMA or an alternative information system, e.g., a desktop application that is used to operate the device 100 for scanning objects by the user, that allows for logging into the system, enabling/disabling the scanning and processing of data, presentation of 3D models and their use in various applications. The user mobile application UMA or alternative information system optionally supports the voice communication, i.e., recognizes the user's voice commands and generates voice notifications.

The above description of the examples of implementation is provided to enable any expert to make or use the present invention. Various modifications of the illustrated examples of implementation are also possible including all such changes, modifications and variations falling within the scope of the appended claims. Thus, the basic principles defined herein may be applied to other examples of implementation without departing from the scope of the invention as defined by the claims. Thus, it is not the intention of the present invention to be limited to the examples of implementation disclosed herein, but to be in accordance with the broadest range consistent with the principles and novel features disclosed herein.

The present solution according to the invention thus offers by the above-mentioned technical means, as indicated in the Figures from **FIG. 1** to **FIG. 8****,** a portable device and system for scanning objects, especially the human figure, using the designed elements and systems intended to automatically collect measurement data concerning the scanned object.

### APPLICATION OF THE INVENTION

The invention can be used in many industries where a 3D model of an object, especially a human figure, can be used. The potential application of the present solution covers, in particular, the clothing industry, including tailoring companies and clothing stores, where thanks to the scan, it will not be necessary to measure clothes, because artificial intelligence will itself assess the shape of the body and select the appropriate clothes.

This solution will also be used in the broadly understood fitness industry, where it will allow one to quickly and accurately measure the progress after exercise by measuring the increase in muscle mass or the progress of fat loss, as well as in the medical industry, especially in centers of therapy of posture defects and rehabilitation and in plastic surgery centers. The solution can also be successfully used in the video game industry, creating 3D models of scanned objects, including, in particular the human figure, e.g., of a player whose figure is then placed in post-production of the game.

### LIST OF REFERENCE DESIGNATIONS

| | | | |
|---|---|---|---|
| **100** | **device for scanning objects** | | |
| 50 | support element | 6 | lower arm |
| 25 | mast | 6b | trolley guide rail |
| 1 | platform | 6f | wire connector |
| 2 | body | 7 | upper arm |
| 2a | gear | 7b | trolley guide rail |
| 2b | bushing | 8 | upper yoke |
| 2c | cable | 8a | lower part of upper yoke |
| 3 | supporting set | 8b | upper part of upper yoke |
| 3a | legs | 8c | screw |
| 3b | screw | 9 | trolley |
| 3c | wheels | 9a | stepper motor |
| 3d | power connector | 9b | toothed belt |
| 3e | magnet | 9c | gear |
| 3f | cross arm | 9d | servo-drive |
| 3g | tubular structure | 10 | lighting mounting connector |
| 4 | rotating arm | 11 | LED lamp |
| 4a | drive assembly | 12 | sensor mounting connector |
| 4b | toothed belt | 13 | sensor set |
| 4c | power distribution board | 14 | AC adapter |
| 4d | arm drive controller | 15 | presence sensor |
| 4e | wire harness | 16 | cable carrier |
| 4f | wire connector | 17 | primary control subsystem |
| 4g | magnetic field sensor | 18 | secondary control subsystem |
| 4h | counterbalance | | |
| 5 | lower yoke | | |
| 5a | upper part of lower yoke | | |
| 5b | lower part of lower yoke | | |
| 5c, 5d | screw | | |

| | | | |
|---|---|---|---|
| **200** | **system for scanning objects** | | |
| P | power subsystem | WAN | wide area network |
| AC | AC adapter / battery | COS | information system |
| DC | DC / DC converter | UMA | user mobile application |
| C | control subsystem | UMD | user mobile device |
| DA | arm drive subsystem | EXT | external information systems |
| DT | trolley motion subsystem | CA | computing application |
| DH | head tilting subsystem | DB | database |
| DL | lighting subsystem | AD | data storage |
| IA | web application | E | external services |
| S | sensor subsystem | | |
| W | communication subsystem | | |

## Claims

1. A portable device (100) for scanning objects having at least one mast (25) with sensor sets (13), connected in its lower part to a support element (50) designed to place the scanned object above it, wherein the mast (25) is situated substantially vertically with respect to the support element (50) comprising a rotating arm (4), a platform (1) and a body (2), wherein the mast (25) is connected to the rotating arm (4) situated horizontally with respect to the mast (25) and connected to the body (2), on which the platform (1) designed to place the scanned object on it is located, and having a supporting set (3) and a control unit for controlling the portable device (100), wherein the mast (25) is shaped such that it rotates with the rotating arm (4) around the stationary platform (1) and the stationary supporting set (3), wherein at least one mast (25) comprises at least one mast lower arm (6) and at least one mast upper arm (7) articulated with each other by means of an upper yoke (8) forming a tilting structure of the mast (25), in such a way that the mast upper arm (7) is tilted in direction to the rotating arm (4) with respect to the mast lower arm (6) and mast lower arm (6) is tilted in direction away from the rotating arm (4) with respect to the mast upper arm (7), wherein the mast lower arm (6) in its lower part is detachably connected to the rotating arm (4) by means of a lower yoke (5).

2. The portable device (100) according to claim 1, **characterized in that** it has a modular structure consisting of at least two modules comprising a first and a second module, wherein the first module has a mast (25) comprising at least one mast lower arm (6) and at least one mast upper arm (7) articulated with each other by means of an upper yoke (8) and the second module has the platform (1) with the body (2) and the rotating arm (4).

3. The portable device (100) according to claim 1, **characterized in that** a tilting structure of the mast (25) allows the mast lower arm (6) to be tilted in direction away from the rotating arm (4) with respect to the mast upper arm (7) within the range of at least 0 degrees to +45 degrees, and the mast upper arm (7) to be tilted in direction to the rotating arm (4) with respect to the mast lower arm (6) within the range of at least -45 degrees to 0 degrees.

4. The portable device (100) according to claim 1, **characterized in that** the sensor set (13) on the mast (25) is mounted by means of a sensor mounting connector (12) on a trolley (9), which, by means of a stepper motor (9a), moves longitudinally in a linear manner, respectively, along the mast lower arm (6) and the mast upper arm (7) to obtain a given number of positions in relation to the scanned object.

5. The portable device (100) according to claim 4, **characterized in that** the sensor set (13) mounted on the sensor mounting connector (12) has the ability to adjust the tilt angle by means of a servo-drive (9d), thus adjusting to the scanned object in the range of at least +/- 45 degrees.

6. The portable device (100) according to claim 5, **characterized in that** the sensor set (13) includes sensors enabling to collect data concerning the scanned object, in particular LIDAR sensors and/or magnetic sensors and/or cameras and/or smartphones equipped with cameras.

7. The portable device (100) according to claim 1 to 4, **characterized in that** a presence sensor (15) is mounted on the mast lower arm (6) to detect the presence of the scanned object on the platform (1).

8. The portable device (100) according to claim 1 or 7, **characterized in that** along the mast lower arm (6) and/or along the mast upper arm (7) a LED bar adjacent directly to the mast arms, respectively, is integrated for lighting up the scanned object, and/or along the mast lower arm (6) and/or along the mast upper arm (7) there is at least one set of LED lamps (11) not adjacent to the mast arms (6) and/or (7), respectively, fixed to the mentioned arms by means of a lighting mounting connector (10).

9. The portable device (100) according to claim 1, **characterized in that** the supporting set (3) is configured in such a way that it allows to change the size of a support surface by means of extendable legs (3a) moving relative to an extendable cross arm (3f) and it allows to change the spacing of the legs (3a) by means of the extendable cross arm (3f) from the tubular structure (3g), wherein the extension of the legs (3a) and/or the extension of the cross arm (3f) is blocked by means of at least one screw (3b).

10. A system (200) for scanning objects, **characterized in that** it comprises the portable device (100) for scanning objects according to any one of the preceding claims 1 to 9, wherein the portable device (100) is equipped with the following subsystems: a control subsystem (C), a power subsystem (P), a communication subsystem (W), a sensor subsystem (S) and an arm drive subsystem (DA), wherein the control subsystem (C) interacts with the power subsystem (P) containing converters (DC) and an AC adapter and/or a battery (AC), wherein between the control subsystem (C) and the power subsystem (P), there is an interaction with the above-mentioned subsystems: the communication subsystem (W), the sensor subsystem (S) and the arm drive subsystem (DA), wherein the mentioned portable device (100) is connected via a communication subsystem (W) to a wide area network (WAN) through which it interacts with an information system (COS), external information systems (EXT) and user mobile devices (UMD) and/or an alternative system.

11. The system (200) according to claim 10, **characterized in that** the mentioned portable device (100) is additionally equipped with the following subsystems: a trolley motion subsystem (DT), a head tilting subsystem (DH) and a lighting subsystem (DL), and those subsystems interact with the control subsystem (C) and the power subsystem (P).

12. The system (200) according to claim 10, **characterized in that** the information system (COS) has a web application (IA), a computing application (CA), a database (DB) and a data storage (AD).

13. The system (200) according to claim 10, **characterized in that** the external information systems (EXT) have, external payment processing systems and social media systems.

14. The system (200) according to claim 10, **characterized in that** it interacts with the user mobile device (UMD) and/or an alternative system configured to manual control or voice control of the portable device (100) for scanning objects and to obtain optional voice notifications and/or to preview the obtained scanned object based on a user mobile application (UMA) installed on the user mobile device (UMD) or a desktop computer.

15. The system (200) according to claim 10, **characterized in that** it has the ability to switch off selected subsystems involved in generating a 3D model such that a user operating the portable device (100) for scanning objects obtains the scanned object of lower quality correspondingly faster by using the user mobile application (UMA) installed on the user mobile device (UMD).

## Patentansprüche

1. Tragbares Gerät (100) zum Scannen von Objekten aufweisend mindestens einen Mast (25) mit Sensorsätzen (13), der in seinem unteren Teil mit einem Trägerelement (50) verbunden ist, das dazu vorgesehen ist, das gescannte Objekt darüber zu platzieren, wobei der Mast (25) zu dem Trägerelement (50), das einen Schwenkarm (4), eine Plattform (1) und einen Körper (2) umfasst, im Wesentlichen vertikal angeordnet ist, wobei der Mast (25) mit dem horizontal zum Mast (25) angeordneten und mit dem Körper (2) verbundenen Schwenkarm (4) verbunden ist, und auf dem Körper (2) sich die Plattform (1) zum Ablegen des gescannten Objekts befindet, und der Schwenkarm (4) einen Stützsatz (3) und eine Steuereinheit zur Steuerung des tragbaren Geräts (100) aufweist, wobei der Mast (25) so ausgebildet ist, dass er sich mit dem Schwenkarm (4) um die stationäre Plattform (1) und den stationären Stützsatz (3) dreht, wobei mindestens ein Mast (25) mindestens einen Mastunterarm (6) und mindestens einen Mastoberarm (7) umfasst, die über ein Oberjoch (8) gelenkig miteinander verbunden sind und eine Kippstruktur des Masts (25) bilden, derart dass der Mastoberarm (7) gegenüber dem Mastunterarm (6) in Richtung zum Schwenkarm (4) und der Mastunterarm (6) gegenüber dem Schwenkarm (4) weg am Mastoberarm (7) geneigt ist, wobei der Mastunterarm (6) in seinem unteren Teil über ein Unterjoch (5) mit dem Schwenkarm (4) lösbar verbunden ist.

2. Tragbares Gerät (100) nach Anspruch 1, **dadurch gekennzeichnet, dass** es einen modularen, aus mindestens zwei Modulen bestehenden Aufbau aufweist, dass ein erstes Modul und ein zweites Modul umfasst, wobei das erste Modul einen Mast (25) aufweist, der mindestens einen Mastunterarm (6) und mindestens einen Mastoberarm (7) enthält, die über ein Oberjoch (8) gelenkig miteinander verbunden sind, und das zweite Modul die Plattform (1) mit dem Körper (2) und dem Schwenkarm (4) aufweist.

3. Tragbares Gerät (100) nach Anspruch 1, **dadurch gekennzeichnet, dass** eine Kippstruktur des Masts (25) ermöglicht, den Mastunterarm (6) in Richtung in Bezug auf den Mastoberarm (7) vom Schwenkarm (4) weg im Bereich von mindestens 0 Grad bis +45 Grad und den Mastoberarm (7) in Bezug auf den Mastunterarm (6) in Richtung zu Schwenkarm (4) in Bereich von mindestens -45 Grad bis 0 Grad zu neigen.

4. Tragbares Gerät (100) nach Anspruch 1, **dadurch gekennzeichnet, dass** der Sensorsatz (13) am Mast (25) mittels eines Sensormontagesteckers (12) auf einem Trolley (9) montiert ist, der mittels eines Schrittmotors (9a) sich linear in Längsrichtung entlang des Mastunterarms (6) und des Mastoberarms (7) bewegt, um eine bestimmte Anzahl von Positionen in Bezug auf das gescannte Objekt zu erhalten.

5. Tragbares Gerät (100) nach Anspruch 4, **dadurch gekennzeichnet, dass** der am Sensorbefestigungsstecker (12) montierte Sensorsatz (13), die Möglichkeit aufweist, den Neigungswinkel mittels eines Servoantriebs (9d) einzustellen und sich somit dem gescannten Objekt im Bereich von mindestens ±45 Grad anzupassen.

6. Tragbares Gerät (100) nach Anspruch 5, **dadurch gekennzeichnet, dass** der Sensorsatz (13) Sensoren, die Daten über das gescannte Objekt zu sammeln ermöglichen, darstellt, insbesondere LIDAR-Sensoren und/oder Magnetsensoren und/oder Kameras und/oder mit Kameras ausgestattete Smartphones.

7. Tragbares Gerät (100) nach einem der Ansprüche 1 bis 4, **dadurch gekennzeichnet, dass** am Mastunterarm (6) ein Anwesenheitssensor (15) montiert ist, um die Anwesenheit des gescannten Objekts auf der Plattform (1) zu erkennen.

8. Tragbares Gerät (100) nach Anspruch 1 oder 7, **dadurch gekennzeichnet, dass** entlang des Mastunterarms (6) und/oder entlang des Mastoberarms (7) jeweils eine direkt an den Mastarmen angrenzende LED-Leiste zur Beleuchtung des gescannten Objekts eingebaut ist und/oder entlang des Mastunterarms (6) und/oder entlang des Mastoberarms (7) mindestens ein nicht neben an die Mastarmen (6) und/oder (7) angrenzende, mittels eines Beleuchtungsbefestigungsverbinders (10) an den genannten Armen befestigte, Satz von LED-Lampen (11) jeweils angeordnet ist.

9. Tragbares Gerät (100) nach Anspruch 1, **dadurch gekennzeichnet, dass** der Stützsatz (3) so ausgestaltet ist, dass es eine Veränderung der Größe einer Stützfläche ermöglicht, indem verlängerbare Beine (3a) relativ zum verlängerbaren Kreuzstück (3f) bewegt werden und es ermöglicht, den Abstand der Beine (3a) mittels des verlängerbaren Kreuzstücks (3f) von der rohrförmigen Struktur (3g) zu verändern, wobei die Verlängerung der Beine (3a) und/oder der Verlängerung des Kreuzstücks (3f) mittels mindestens einer Schraube (3b) blockiert ist.

10. System (200) zum Scannen von Objekten, **dadurch gekennzeichnet, dass** es das tragbare Gerät (100) zum Scannen von Objekten nach einem der vorhergehenden Ansprüche 1 bis 9 enthält, wobei das tragbare Gerät (100) mit den folgenden Subsystemen ausgestattet ist: ein Steuersubsystem (C), ein Energieversorgungssubsystem (P), ein Kommunikationssubsystem (W), ein Sensorsubsystem (S) und ein Armantriebssubsystem (DA), wobei das Steuersubsystem (C) mit dem ein Umformer (DC) und ein AC-Adapter und/oder ein Akkumulator (AC) enthaltenden Energieversorgungssubsystem (P) interagiert, wobei zwischen dem Steuersubsystem (C) und dem Energieversorgungssubsystem (P) eine Wechselwirkung mit den oben genannten Subsystemen (dem Kommunikationssubsystem (W), dem Sensorsubsystem (S) und dem Armantriebssubsystem (DA) besteht, wobei das genannte tragbare Gerät (100) über ein Kommunikationssubsystem (W) mit einem Weitverkehrsnetz (WAN) verbunden ist, und über WAN es mit einem Informationssystem (COS), externen Informationssysteme (EXT) und mobilen Benutzergeräten (UMD) und/oder einem alternativen System interagiert.

11. Das System (200) nach Anspruch 10, **dadurch gekennzeichnet, dass** das genannte tragbare Gerät (100) zusätzlich mit den folgenden Subsystemen ausgestattet ist: einem Trolley-Bewegungs-Subsystem (DT), einem Kopfneigungs-Subsystem (DH) und einem Beleuchtungs-Subsystem (DL), und diese Subsysteme mit dem Steuersubsystem (C) und dem Energieversorgungssubsystem (P) interagieren.

12. System (200) nach Anspruch 10, **dadurch gekennzeichnet, dass** das Informationssystem (COS) eine Webapplikation (IA), eine Computerapplikation (CA), eine Datenbank (DB) und einen Datenspeicher (AD) aufweist.

13. System (200) nach Anspruch 10, **dadurch gekennzeichnet, dass** die externen Informationssysteme (EXT) externe Zahlungsabwicklungssysteme und Social-Media-Systeme aufweisen.

14. Das System (200) nach Anspruch 10, **dadurch gekennzeichnet, dass** es mit dem mobilen Benutzergerät (UMD) und/oder einem alternativen System interagiert, das zur manuellen Steuerung oder Sprachsteuerung des tragbaren Geräts (100) zum Scannen von Objekten und zu optionalen Sprachbenachrichtigungen und/oder einem Vorschau des erhaltenen gescannten Objekts basierend auf einer mobilen Benutzerapplikation (UMA) konfiguriert ist), die auf dem mobilen Benutzergerät (UMD) oder einem Desktop-Computer installiert ist.

15. Das System (200) nach Anspruch 10, **dadurch gekennzeichnet, dass** es die Fähigkeit aufweist, ausgewählte Subsysteme, die an der Erstellung eines 3D-Modells beteiligt sind, abzuschalten, sodass ein Benutzer, der das tragbare Gerät (100) zum Scannen von Objekten bedient, das gescannte Objekt von unten Qualität entsprechend schneller erhält, indem die auf dem mobilen Benutzergerät (UMD) installierte mobile Benutzerapplikation (UMA) verwendet wird.

## Revendications

1. Dispositif transportable (100) pour scanner d'objets comportant au moins un mât (25) avec des ensembles de capteurs (13), relié dans sa partie inférieure à un élément de support (50) destiné à placer l'objet scanné au-dessus de lui, dans lequel le mât (25) est situé en effet verticalement par rapport à l'élément de support (50) comprenant un bras rotatif (4), une plate-forme (1) et un corps (2), le mât (25) étant relié au bras rotatif (4) situé horizontalement par rapport au mât (25) et relié au corps (2), sur lequel se trouve la plate-forme (1) conçue pour y placer l'objet scanné, et comportant un ensemble de support (3) et une unité de commande pour commander le dispositif transportable (100), dans lequel le mât (25) est formé de telle sorte qu'il tourne avec le bras rotatif (4) autour de la plate-forme fixe (1) et de l'ensemble de support fixe (3), dans lequel au moins un mât (25) comprend au moins un bras inférieur (6) du mât et au moins un bras supérieur (7) du mât articulés entre eux au moyen d'un étrier supérieur (8) formant une structure inclinable du mât (25), de telle manière que le bras supérieur (7) du mât est incliné en direction du bras rotatif (4) par rapport au bras inférieur (6) du mât et le bras inférieur (6) du mât est incliné en direction opposée au bras rotatif (4) par rapport au bras supérieur (7) du mât, le bras inférieur (6) du mât dans sa partie inférieure étant relié de manière amovible au bras rotatif (4) au moyen d'un étrier inférieur (5).

2. Dispositif transportable (100) selon la revendication 1, **caractérisé en ce qu'**il présente une structure modulaire constituée d'au moins deux modules comprenant un premier module et un deuxième module, dans lequel le premier module présente un mât (25) comprenant au moins le bras inférieur (6) du mât et au moins le bras supérieur (7) du mât articulé l'un avec l'autre au moyen d'un étrier supérieur (8) et le deuxième module comporte la plate-forme (1) avec le corps (2) et le bras rotatif (4).

3. Dispositif transportable (100) selon la revendication 1, **caractérisé en ce qu'**une structure inclinable du mât (25) permet au bras inférieur (6) du mât d'être incliné en direction opposée au bras rotatif (4) par rapport au bras supérieur (7) du mât dans une plage d'au moins 0 degrés à +45 degrés, et au bras supérieur (7) du mât d'être incliné en direction du bras rotatif (4) par rapport au bras inférieur (6) du mât dans une plage d'au moins -45 degrés à 0 degrés.

4. Dispositif transportable (100) selon la revendication 1, **caractérisé en ce que** l'ensemble de capteurs (13) sur le mât (25) est monté au moyen d'un connecteur de montage de capteur (12) sur un chariot (9), qui, par au moyen d'un moteur pas à pas (9a), se déplace longitudinalement de manière linéaire, respectivement, le long du bras inférieur (6) du mât et du bras supérieur (7) du mât pour obtenir un nombre donné de positions par rapport à l'objet scanné.

5. Dispositif transportable (100) selon la revendication 4, **caractérisé en ce que** l'ensemble de capteurs (13) monté sur le connecteur de montage de capteur (12) a la capacité de régler l'angle d'inclinaison au moyen d'un servomoteur (9d), s'ajustant ainsi à l'objet scanné dans une plage d'au moins +/- 45 degrés.

6. Dispositif transportable (100) selon la revendication 5, **caractérisé en ce que** l'ensemble de capteurs (13) comprend des capteurs permettant de collecter des données concernant l'objet scanné, notamment des capteurs LIDAR et/ou des capteurs magnétiques et/ou des caméras et/ou des smartphones équipés de caméras.

7. Dispositif transportable (100) selon la revendication 1 à 4, **caractérisé en ce qu'un** capteur de présence (15) est monté sur le bras inférieur (6) du mât pour détecter la présence de l'objet scanné sur la plate-forme (1).

8. Dispositif transportable (100) selon la revendication 1 ou 7, **caractérisé en ce que** le long du bras inférieur (6) du mât et/ou le long du bras supérieur (7) du mât, une barre LED adjacente directement aux bras du mât, respectivement, est intégré pour éclairer l'objet scanné, et/ou le long du bras inférieur (6) du mât et/ou le long du bras supérieur (7) du mât, il y a au moins un ensemble de lampes à LED (11) non adjacentes aux bras du mât (6) et/ou (7), respectivement, fixés aux bras mentionnés au moyen d'un connecteur de montage d'éclairage (10).

9. Dispositif transportable (100) selon la revendication 1, **caractérisé en ce que** l'ensemble de support (3) est configuré de telle manière qu'il permet de modifier la taille d'une surface de support au moyen de pieds extensibles (3a) se déplaçant par rapport à un bras traversable extensible (3f) et permet de modifier l'espacement des pieds (3a) au moyen du bras traversable extensible (3f) par rapport à la structure tubulaire (3g), dans lequel l'extension des pieds (3a) et/ou l'extension du bras traversable (3f) est bloqué au moyen d'au moins une vis (3b).

10. Système (200) de scanner d'objets, **caractérisé en ce qu'**il comprend le dispositif transportable (100) de scanner d'objets selon l'une quelconque des revendications précédentes 1 à 9, dans lequel le dispositif transportable (100) est équipé des sous-systèmes suivants: un sous-système de commande (C), un sous-système de puissance (P), un sous-système de communication (W), un sous-système de capteur (S) et un sous-système d'entraînement de bras (DA), le sous-système de commande (C) interagissant avec le sous-système de puissance (P) contenant des convertisseurs (DC) et un adaptateur secteur et/ou une batterie (AC), dans lequel entre le sous-système de commande (C) et le sous-système de puissance (P), il existe une interaction avec les sous-systèmes mentionnés ci-dessus: le sous-système de communication (W), le sous-système de capteur (S) et le sous-système d'entraînement de bras (DA), le dispositif transportable mentionné (100) étant connecté via le sous-système de communication (W) à un réseau étendu (WAN) à travers lequel il interagit avec un système d'information (COS), systèmes d'information externes (EXT) et appareils mobiles des utilisateurs (UMD) et/ou un système alternatif.

11. Système (200) selon la revendication 10, **caractérisé en ce que** le dispositif transportable mentionné (100) est en outre équipé des sous-systèmes suivants: un sous-système de mouvement du chariot (DT), un sous-système d'inclinaison de la tête (DH) et un sous-système d'éclairage (DL), et ces sous-systèmes interagissent avec le sous-système de contrôle (C) et le sous-système de puissance (P).

12. Système (200) selon la revendication 10, **caractérisé en ce que** le système d'information (COS) comporte une application web (IA), une application informatique (CA), une base de données (DB) et un stockage de données (AD).

13. Système (200) selon la revendication 10, **caractérisé en ce que** les systèmes d'information externes (EXT) disposent de systèmes externes de traitement des paiements et de systèmes de médias sociaux.

14. Système (200) selon la revendication 10, **caractérisé en ce qu'**il interagit avec l'appareil mobile de l'utilisateur (UMD) et/ou un système alternatif configuré pour une commande manuelle ou vocale du dispositif transportable (100) pour scanner des objets et pour obtenir des notifications vocales facultatives et/ou pour prévisualiser l'objet scanné obtenu sur la base d'une application mobile de l'utilisateur (UMA) installée sur l'appareil mobile de l'utilisateur (UMD) ou sur un ordinateur de bureau.

15. Système (200) selon la revendication 10, **caractérisé en ce qu'**il a la capacité de désactiver des sous-systèmes sélectionnés impliqués dans la génération d'un modèle 3D de telle sorte qu'un utilisateur utilisant le dispositif transportable (100) pour scanner des objets obtienne l'objet scanné de qualité inférieure d'autant plus rapide en utilisant l'application mobile de l'utilisateur (UMA) installée sur l'appareil mobile de l'utilisateur (UMD).
